# EUROPEAN PATENT APPLICATION

(11) **EP 2 417 933 A2**
(43) Date of publication of application: **15.02.2012**
(21) Application number: 11177089.7
(22) Date of filing: 10.08.2011
(51) Int. Cl.: A61F 2/14

(54) **Bio-sheet for eye tissue repair**

(30) Priority: 11.08.2010 CN 201020289394 U
(71) Applicant: Body Organ Biomedical Corp., Taiwan (CN)
(72) Inventor: Lai, Horng-Ji, 106 Taipei (TW); Lin, Feng-Huei, 106 Taipei City (TW); Lin, Chien-Cheng, 111 Taipei City (TW); Lin, Shang-Ming, 420 Taichung County (TW); Chang, Yu-Chung, 710 Tainan County (TW)
(74) Representative: Winkler, Andreas Fritz Ernst

(57) **Abstract**

A bio-sheet applied on the eye tissue repair is disclosed. The bio-sheet comprises a biodegradable substrate formed of fish scales and has an epithelial cell layer formed thereon, wherein the fish scales are bony scales.

## Description

### Field of the invention

The present invention pertains to a bio-sheet particularly to a bio-sheet for ophthalmologic tissue repair.

### Description of the prior art:

Most of human corneal diseases can be remedied by healing medicine. However, once medicine is ineffective, the patient may need a corneal transplantation to recover eyesight. For corneal transplant surgery, a crucial part of it is what material can be used to replace the original damaged cornea.

Generally, the material for using in corneal transplant can be classified as autograft, allograft, and xenograft in accordance with the source If the corneal transplant material comes from the patient himself or herself, it is called autotransplantation or autograft approach. The approach comprises the steps of taking the limbal stem cells from patient's healthy eye,culturing the eye limbal stem cells and then transplanting the cultured eye limbal stem cells back to the patient's damaged cornea so as to repair the damaged portion of cornea. However, such restoration may remove too much corneal limbal stem cells, leaving the other healthy eye damaged during the surgery. In addition, the patient must have the other eye function properly and ensure no danger after surgery. Therefore, the practical application is low if the portion of cornea to be transplanted is too big because it may affect the healthy eye.

In comparison with the autotransplantation, if the material for the corneal transplant does not come from the patient himself/herself but a dornor it is called allograft. In fact, due to the problems of inadequate source of donors and immunnological rejection often lead to the autotransplantation cannot meet the needs of patients.

For the xenograft using amniotic membrane as the material of cornea is concerned, amniotic membrane transplantation has the benefits of inhibiting the inflammation affected area and promoting epidermal growth, but because there are no stem cells in amniotic membrane and the recovering mechanism completely rely on the patient's own remaining cells grow back. As a result, the restoration is poor if the damaged area of patient is a little big. In addition, the source of amniotic membrane transplantation is restricted because the amniotic membrane comes from the human C-section or from fresh placenta of natural delivery sterile delivery, and if the sterilization process is not complete, the infectious problem could not be ruled out.

Asides from aforementioned transplantation, there is also including using the production of the biomedical polymer as a graft materiall. One of the shortcomings of using the biomedical polymer as corneal graft is the processes from monomer polymerization to the final product whose reaction conditions are difficult to handle. The final products may vary from batch to batch. In addition, the graft material is used in ophthalmic surgery, so that the transparency of graft material will be main factor to determine whether the graft material meet the ophthalmic conditions. However, the biomedical polymer after several processes, the properties of the materials often different, making the material transparency various.

Hence an object of the present is to develop a corneal repair material so as to overcome forgoing problems.

### Summary of the Invention:

An object of the present invention is to provide a bio-sheet applied on the eye-vision repair.

The other object of the present invention is to substitute the tissue repair of biomaterial currently.

Still another object of the present invention is to provide a bio-sheet for those patients whose corneal tissue is partially damaged or even severely damaged, particularly for those patients whose corneal with a little or lack of limbal stem cells.

The present invention disclosed a bio-sheet applied on the eye tissue repair The bio-sheet comprises a biodegradable substrate formed of fish scales and has an epithelial cell layer formed thereon, wherein the fish scales are bony scales.
1. The biodegradable substrate is transparency and with a tensile strength between about 0.5-50 Mpa. The bio-sheet has a diameter between about 5-20 mm and central thickness between 0.03-1 mm. The epithelial cell layer is formed of epithelial cell retaining the function and morphology as the prototype cells. Preferably, the epithelial cells is selected from mucosal cells, hair root cells and conjunctiva epithelial cells and the fish scales are selected from cycoloid scales and ctenoid scales.

### Brief description of the drawings

The foregoing aspects and many of the attendant advantages of this invention will become more readily appreciated as the same becomes better understood by reference to the following detailed description, when taken in conjunction with the accompanying drawings, wherein:

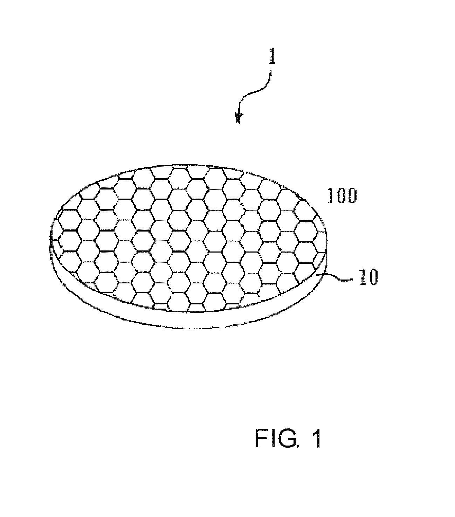

FIG.1 illustrates a structure of the bio-sheet according to the present invention.

### Description of the Preferred Embodiment

Referring to FIG.1, a bio-sheet 1 using for corneal epithelial cells repair is shown in accordance with the first embodiment of the present invention. The bio-sheet 1 is a biodegradable substrate 10 formed of fish scales, having an epithelial cell layer 100 formed thereon.

The size of the biodegradable substrate 10 used depends on the injured position and area thereof. Generally, a biodegradable substrate 10 having a diameter of about 5-20 mm, central thickness of about 0.03-1 mm and tensile strength of about 0.5-50 Mpa is preferred,

In the present invention, the biodegradable substrate 10 contains a bound of collagen, which can promote the proliferation of healthy cells around the wound, while providing structural support of tissue growth so that cells can attach and grow to form new tissue. More importantly, the collagen does not induce residual problems in the human body. It is because new implanted collagen is predicted absorptive by the body's enzymes through the mechanism of decomposition, proliferation, and repair that allows the collagen in body function more favorable.

The biodegradable substrate 10 may be a disc-shaped. Surely, the biodegradable substrate 10 may be a transparent substrate but with a shape in accordance with the predetermined requirement. Furthermore, the transparent substrate 10 may also have porous formed therein. In the meanwhile, the biodegradable substrate 10 formed of fish scales, which may be bony scales selected from the cycloid scales or ctenoid scales in osteichthyes. Preferably, the fish scales are treated by processes of acelluralization and decalcification.

In a preferred embodiment, in order to ensure the growth and activity of the epithelial cells in the epithelial cell layer 100 of the biodegradable substrate 10, a bio-sheet 1 demanded several environmental factors such as a temperature range of about 37 °C, the carbon dioxide content of about 5% and in one atmospheric pressure.

Preferably, the source of the epithelial cell layer 100 may be selected from the group consisting of mucosal cells, hair root cells and conjunctival epithelial cells. To retain the original function and activity of epithelial cells in the epithelial cells layer 100 as they were, the cells are prototype cells. The mucosal cells are the mouth lining cells or other equivalent body's portions of the patient herself/himself and then cell cultivated so as to avoid immunlogical rejection. The epithelial cells selected have to compatible to the patient.

The bio-sheet 1 can be used to repair the front half portion, all, or partial of the damaged eye tissue wherein the front half portion of the eye tissue is the epithelial tissue, Bowman's membrane and corneal stromal tissue etc.

The benefits of the present invention are as follows:
(1). Fish scales are easy to obtain, no restriction on source.
(2). No exchange of the disease between fish and human is reported at least in so far and thus the biodegradable substrate 10 made of fish scales used for cornea is safety.
(3). Fish scales are natural materials for biographic used materials.
The prepared processes are simple and easier to control the processing conditions.
(4). The main component of fish scales is collagen, in a favor of cornea tissue repair.
(5). Fish scales with a complete microstructure, good mechanical strength and moreover, decalcification fish scales are a transparency material and the calcification risk after implanted into organisms is low.
(6). Fish scales with epithelial cells are helpful to recover the eyesight of patients whose limbal stem cells were damaged.

As is understood by a person skilled in the art, the foregoing preferred embodiments of the present invention are illustrated of the present invention rather than limiting of the present invention. It is intended to cover various modifications and similar arrangements included within the spirit and scope of the appended claims, the scope of which should be accorded the broadest interpretation so as to encompass all such modifications and similar structures.

## Claims

1. A bio-sheet, using in eye tissue repair, comprising:
a biodegradable substrate formed of fish scales having a epithelial cell layer formed thereon, wherein said fish scales are bony scales.

2. The bio-sheet according to the claim 1 wherein said biodegradable substrate is a transparent substrate.

3. The bio-sheet according to the claim 1 wherein said biodegradable substrate has a tensile strength between about 0.5 MPa to 50 MPa.

4. The bio-sheet according to the claim 1 wherein said biodegradable substrate has a diameter between about 5 to 20 mm.

5. The bio-sheet according to the claim 1 wherein said biodegradable substrate has a central thickness between about 0.03 to 1 mm.

6. The bio-sheet according to the claim 1 wherein said epithelial cell layer retains morphology and function as epithelial cells had.

7. The bio-sheet according to the claim 1 wherein said epithelial cell layer is selected from the group consisting of mucosal cells, hair root cells and conjunctiva epithelial cells.

8. The bio-sheet according to the claim 1 wherein said fish scales are selected from cycoioid scales and ctenoid scales.
